# EUROPEAN PATENT SPECIFICATION

(11) **EP 3 770 154 B1**
(45) Date of publication and mention of the grant of the patent: **19.02.2025**
(21) Application number: 19771646.7
(22) Date of filing: 06.03.2019
(51) Int. Cl.: C07D 319/12, C07B 61/00

(54) **GLYCOLIDE PRODUCTION METHOD**
GLYKOLIDHERSTELLUNGSVERFAHREN
PROCÉDÉ DE PRODUCTION DE GLYCOLIDE

(30) Priority: 20.03.2018 JP 2018052289; 20.03.2018 JP 2018052293
(43) Date of publication of application: 27.01.2021
(73) Proprietor: Kureha Corporation, Chuo-ku Tokyo 103-8552 (JP)
(72) Inventor: YAMAJI, Haruyasu, Tokyo 103-8552 (JP); YAMADOI, Yuta, Tokyo 103-8552 (JP); SUZUKI, Yoshinori, Tokyo 103-8552 (JP); ONO, Toshihiko, Tokyo 103-8552 (JP)
(74) Representative: Novagraaf Group
(86) International application number: PCT/JP2019/008921
(87) International publication number: WO 2019/181516

(56) References cited:
- EP-A1- 1 553 076
- WO-A1-02/14303
- JP-A- 2000 119 269
- JP-A- H09 328 481
- JP-B1- S4 919 117
- JP-B2- S 482 228

## Description

### Technical Field

The present invention relates to a method for producing glycolide.

### Background Art

Polyglycolic acid is a resin material that excels in biodegradability, gas barrier properties, and strength, and is used in a wide range of technical fields such as in sutures, artificial skin, and other polymer materials for medical purposes, bottles, films, and other packaging materials, and resin materials for various industrial products such as injection molded products, fibers, vapor deposition films, and fishing lines.

Such polyglycolic acids are required to have a high degree of polymerization according to the application. A polyglycolic acid with a high degree of polymerization can be produced by a method of subjecting glycolide to ring-opening polymerization. Furthermore, a reduction of the production costs of polyglycolic acid is demanded, and there is also a demand for the mass production of glycolide used as a raw material, that is, there is a demand to enable the production of glycolide at a high production rate.

Glycolide can be produced through 1) subjecting glycolic acid to dehydrating polycondensation to obtain a glycolic acid oligomer (dehydrating polycondensation), and 2) depolymerizing the obtained glycolic acid oligomer (depolymerization).

Examples of methods for producing glycolide with high yield or efficiently include a method of carrying out a depolymerization reaction of a glycolic acid oligomer in the presence of tin octylate as a catalyst (for example, see Patent Document 1), and a method of carrying out a depolymerization reaction of a glycolic acid oligomer in the presence of titanium alkoxide (Ti(OH)₄) solution in methoxyethanol as a catalyst (for example, see Patent Document 2).

In addition, a method is known in which an aqueous solution of 70% glycolic acid is subjected to dehydrating polycondensation while being gradually heated to 150°C in a reaction vessel made of titanium, and the obtained glycolic acid oligomer is heated under reduced pressure to perform solid-phase depolymerization (for example, see Patent Document 3).

### Citation List

### Patent Document

Patent Document 1: JP 2015-145345 A
Patent Document 2: JP 2013-535433 T
Patent Document 3: JP 2000-119269 A

### Summary of Invention

### Technical Problem

However, with the glycolide production methods described in Patent Documents 1 and 2, the production rate of glycolide is insufficient. Moreover, while the glycolide production method described in Patent Document 3 can be used to favorably produce glycolide, from the perspective of reducing the cost to produce polyglycolic acid having a high degree of polymerization, there is a demand to further improve the production rate of the glycolide that is used as a raw material.

In light of the foregoing, an object of the present invention is to provide a glycolide production method that can sufficiently increase the production rate of glycolide.

### Solution to Problem

The glycolide production method of the present invention includes: adding metal titanium to an aqueous glycolic acid solution; subjecting glycolic acid contained in the aqueous glycolic acid solution to which the metal titanium is added, to dehydrating polycondensation to obtain a glycolic acid oligomer; and heating and depolymerizing the glycolic acid oligomer to obtain glycolide under the existence of a titanium ion generated from the metal titanium; wherein the metal titanium is a titanium powder, and an addition amount of the metal titanium is from 5 ppm to 1000 ppm relative to the total mass of the glycolic acid.

### Advantageous Effects of Invention

According to the present invention, a glycolide production method capable of sufficiently increasing the production rate of glycolide can be provided.

### Description of Embodiments

The present inventors focused on the addition of metal titanium as a catalyst. Ordinarily, a catalyst is typically added in the depolymerization to increase the rate of production of glycolide. The depolymerization is preferably carried out in an organic solvent from the perspective of being able to stably produce glycolide in large quantities. However, metal titanium cannot be dissolved in an organic solvent even when added in the depolymerization, and thus it is not possible to effectively exhibit the action of the metal titanium.

In contrast, in the present invention, metal titanium is added to the aqueous glycolic acid solution used in the dehydrating polycondensation. Metal titanium typically does not dissolve in an aqueous solution, but since the pH of an aqueous glycolic acid solution is low, the metal titanium favorably dissolves in the aqueous glycolic acid solution, and an aqueous glycolic acid solution containing titanium ions can be obtained. On the other hand, when a known titanium-based catalyst such as a titanium alkoxide or a titanium carboxylate described in Patent Document 2 is added to an aqueous glycolic acid solution, the titanium-based catalyst is hydrolyzed and precipitated, and does not function as a catalyst.

In the present description, it is thought that by performing a dehydrating polycondensation using an aqueous glycolic acid solution containing eluted titanium ions, the rate of the dehydrating polycondensation reaction can be increased by the catalytic action of the titanium ions. In addition, it is thought that unlike known titanium-based catalysts such as titanium carboxylates and titanium alkoxides, titanium ions are not affected by ligands, and therefore tend to be highly dispersed in the obtained glycolic acid oligomer. It is also thought that by performing the depolymerization using such a glycolic acid oligomer, the rate of the depolymerization reaction can be effectively increased by the catalytic action of the titanium ions. In particular, titanium (titanium ions) in a highly active state can be supplied into the glycolic acid oligomer by adding metal titanium to the aqueous glycolic acid solution and supplying the metal titanium into the glycolic acid oligomer. It is also thought that as a result, the metal titanium, even added in a low amount, action as a catalyst is easily obtained, and the production rate of glycolide can be dramatically increased.

Furthermore, the addition of metal titanium can also be performed by "heating the aqueous glycolic acid solution in a reaction vessel of which at least the inner surface is constituted by titanium or an alloy thereof, and maintaining the solution at a temperature lower than the boiling point." Consequently, the production rate of glycolide can be dramatically increased.

The reason for this is thought to be as follows. That is, since the pH of the aqueous glycolic acid solution is low, the titanium is eluted into the aqueous glycolic acid solution from the inner surface of the reaction vessel while the aqueous glycolic acid solution is maintained at a temperature lower than the boiling point. It is thought that by carrying out the dehydrating polycondensation using an aqueous glycolic acid solution containing eluted titanium ions in this manner, the rate of the dehydrating polycondensation reaction is increased by the catalytic action of the titanium ions. Furthermore, titanium ions can be favorably dispersed in the obtained glycolic acid oligomer. It is also thought that by performing the depolymerization using such a glycolic acid oligomer, the rate of depolymerization reaction is increased by the catalytic action of the titanium ions. It is further thought that as a result, the production rate of glycolide is dramatically increased.

**In** this manner, titanium ions eluted from the reaction vessel are easily and favorably dispersed in the aqueous glycolic acid solution and in the glycolic acid oligomer that is produced, and therefore catalytic action can be effectively obtained.

### 1. Glycolide Production Method

The glycolide production method according to an embodiment of the present invention includes: 1) adding metal titanium to an aqueous glycolic acid solution (metal titanium addition), 2) subjecting glycolic acid contained in the aqueous glycolic acid solution to which the metal titanium is added, to dehydrating polycondensation to obtain a glycolic acid oligomer (dehydrating polycondensation), and 3) heating and depolymerizing the obtained glycolic acid oligomer to obtain glycolide (depolymerization), whereby the heating and depolymerization is carried out under the existence of a titanium ion generated from the metal titanium; wherein the metal titanium is a titanium powder, and an addition amount of the metal titanium is from 5 ppm to 1000 ppm relative to the total mass of the glycolic acid.

### Step 1) Metal Titanium Addition

Metal titanium is added to an aqueous glycolic acid solution. Through this, at least a portion of the metal titanium is dissolved in the aqueous glycolic acid solution.

The aqueous glycolic acid solution is an aqueous solution containing glycolic acid. The glycolic acid may be an ester (for example, a lower alkyl ester), a salt (for example, a sodium salt), or the like.

The content of glycolic acid with respect to the total mass of the aqueous glycolic acid solution is, for example, preferably from 1 mass% to 99 mass%, and more preferably from 50 mass% to 90 mass%.

The metal titanium is titanium that may contain other components than titanium, but from the perspective of suppressing unnecessary reactions of other components than titanium, the content of the other components than titanium is preferably 10 mass% or less. The form of the metal titanium is a titanium powder

The average particle size of the titanium powder is not particularly limited, but, for example, from the perspective of facilitating uniform dispersion in the aqueous glycolic acid solution, the titanium powder has an average particle size of 100 µm or less, and more specifically, the average particle size is preferably from 1 µm to 100 µm, and more preferably 50 µm or less. The average particle size of the titanium powder can be measured as an arithmetic mean of the volume average particle size distribution using a particle size distribution measurement device.

The addition amount of the metal titanium is from 5 ppm to 1000 ppm, preferably from 5 ppm to 400 ppm, and more preferably from 10 ppm to 50 ppm. When the addition amount of the metal titanium is a certain amount or greater, the rate of the dehydrating polycondensation reaction of the glycolic acid and the rate of the depolymerization reaction of the glycolic acid oligomer are easily increased, and as a result, the production rate of glycolide is easily increased. However, when the addition amount thereof is too high, side reactions tend to increase. When the addition amount of the metal titanium is a certain amount or less, the remaining amount of undissolved metal titanium is easily reduced, thereby facilitating a reduction in recovery costs. However, when the addition amount thereof is too low, it becomes difficult to obtain catalytic action.

From the perspective of facilitating uniform dispersion of the metal titanium, the metal titanium may be added while heating the aqueous glycolic acid solution. From a similar perspective, the metal titanium may be added while stirring the aqueous glycolic acid solution.

The metal titanium addition is performed before step 2).

The aqueous glycolic acid solution is an aqueous solution containing glycolic acid. The glycolic acid may be an ester (for example, a lower alkyl ester), a salt (for example, a sodium salt), or the like.

The content of glycolic acid with respect to the total mass of the aqueous glycolic acid solution is, for example, preferably from 1 mass% to 99 mass%, and more preferably from 50 mass% to 90 mass%.

As the aqueous glycolic acid solution, a purified product (high purity grade) having a low content of impurities such as organic material and metal ions is preferably used in order to facilitate production of high purity glycolide.

The method of reflux of the aqueous glycolic acid solution is not particularly limited, and a method such as stirring or circulation can be employed. In the case of stirring, the stirring speed is not particularly limited as long as air bubbles are not mixed in.

### Step 2) Dehydrating Polycondensation

The aqueous glycolic acid solution obtained in step 1) described above is heated to subject the glycolic acid to dehydrating polycondensation, and a glycolic acid oligomer is obtained. More specifically, the aqueous glycolic acid solution is heated until the distillation of low molecular weight substances such as water or alcohol is substantially completed, and the glycolic acid is subjected to polycondensation.

The heating temperature during the dehydrating polycondensation reaction (dehydrating polycondensation temperature) is preferably from 50°C to 300°C, more preferably from 100°C to 250°C, and even more preferably from 140°C to 230°C.

After the dehydrating polycondensation reaction is completed, the produced glycolic acid oligomer can be used as is as a raw material for step 3)
(depolymerization) described below.

The obtained glycolic acid oligomer contains titanium ions dissolved in the aforementioned step 1). Whether titanium is contained in the glycolic acid oligomer can be confirmed by, for example, ion chromatography (IC), ICP emission spectroscopy, and absorptiometric analysis.

The weight average molecular weight (Mw) of the obtained glycolic acid oligomer is preferably from 1000 to 100000, and more preferably from 10000 to 100000, from the perspective of glycolide yield. The weight average molecular weight (Mw) can be measured by gel permeation chromatography (GPC).

From the perspective of the yield of glycolide for the depolymerization reaction, the melting point (Tm) of the obtained glycolic acid oligomer is, for example, preferably 140°C or higher, more preferably 160°C or higher, and even more preferably 180°C or higher. The upper limit of the melting point (Tm) of the glycolic acid oligomer is, for example, 220°C. Here, the melting point (Tm) of the glycolic acid oligomer can be measured from the endothermic peak temperature when the glycolic acid oligomer is heated at a rate of 10°C/min in an inert gas atmosphere using a differential scanning calorimeter (DSC).

### Step 3) Depolymerization

The glycolic acid oligomer obtained in step 2) described above is heated and depolymerized to obtain glycolide.

The depolymerization may be any of solid phase depolymerization, melt depolymerization, or solution depolymerization, but solution depolymerization is preferable from the perspective of being able to stably produce glycolide in large quantities. That is, preferably, the glycolic acid oligomer is heated in an organic solvent and depolymerized to obtain glycolide.

First, the glycolic acid oligomer is added to an organic solvent to be described below, and heated under normal pressure or under reduced pressure to dissolve the glycolic acid oligomer in the organic solvent.

### Organic Solvent

From the perspective of appropriately increasing the depolymerization reaction temperature and facilitating an increase in the production rate of glycolide, the organic solvent is preferably a high boiling point organic solvent having a boiling point of from 230°C to 450°C, preferably from 235°C to 450°C, more preferably from 255°C to 430°C, and even more preferably from 280°C to 420°C.

Examples of such high boiling point organic solvents include aromatic dicarboxylic acid diesters, aromatic carboxylic acid esters, aliphatic dicarboxylic acid diesters, polyalkylene glycol diethers, aromatic dicarboxylic acid dialkoxyalkyl esters, aliphatic dicarboxylic acid dialkoxyalkyl esters, polyalkylene glycol diesters, and aromatic phosphoric acid esters. Among these, aromatic dicarboxylic acid diesters, aromatic carboxylic acid esters, aliphatic dicarboxylic acid diesters, and polyalkylene glycol diethers are preferable, and from the perspective of being less likely to cause thermal degradation, a polyalkylene glycol diether is more preferable.

As the polyalkylene glycol diether, a polyalkylene glycol diether represented by Formula (1) below is preferable.
[Chemical Formula 1]

X-O-(-R-O-)p-Y (1)

**In** Formula (1), R denotes a methylene group or a linear or branched alkylene group having from 2 to 8 carbons. X and Y each denote an alkyl group or an aryl group having from 2 to 20 carbons, and p is an integer from 1 to 5. When p is 2 or greater, the plurality of R moieties may be mutually the same or different.

Examples of polyalkylene glycol diethers include polyalkylene glycol dialkyl ether, polyalkylene glycol alkyl aryl ether, and polyalkylene glycol diaryl ether.

Examples of polyalkylene glycol dialkyl ethers include diethylene glycol dialkyl ethers such as diethylene glycol dibutyl ether, diethylene glycol dihexyl ether, diethylene glycol dioctyl ether, diethylene glycol butyl-2-chlorophenyl ether, diethylene glycol butylhexyl ether, diethylene glycol butyloctyl ether, and diethylene glycol hexyloctyl ether; triethylene glycol dialkyl ethers such as triethylene glycol diethyl ether, triethylene glycol dipropyl ether, triethylene glycol dibutyl ether, triethylene glycol dihexyl ether, triethylene glycol dioctyl ether, triethylene glycol butyloctyl ether, triethylene glycol butyldecyl ether, triethylene glycol butylhexyl ether, and triethylene glycol hexyloctyl ether; polyethylene glycol dialkyl ethers such as tetraethylene glycol diethyl ether, tetraethylene glycol dipropyl ether, tetraethylene glycol dibutyl ether, tetraethylene glycol dihexyl ether, tetraethylene glycol dioctyl ether, tetraethylene glycol butylhexyl ether, tetraethylene glycol butyloctyl ether, tetraethylene glycol hexyloctyl ether, and other such tetraethylene glycol dialkyl ethers; and polypropylene glycol dialkyl ethers for which the ethyleneoxy group in the polyalkylene glycol dialkyl ether is substituted with a propyleneoxy group, and polybutylene glycol dialkyl ethers for which the ethyleneoxy group in the polyalkylene glycol dialkyl ether is substituted with a butyleneoxy group.

Examples of polyalkylene glycol alkyl aryl ethers include diethylene glycol butylphenyl ether, diethylene glycol hexylphenyl ether, diethylene glycol octylphenyl ether, triethylene glycol butylphenyl ether, triethylene glycol hexylphenyl ether, triethylene glycol octylphenyl ether, tetraethylene glycol butylphenyl ether, tetraethylene glycol hexylphenyl ether, tetraethylene glycol octylphenyl ether, and polyethylene glycol alkyl aryl ethers for which some of the hydrogen atoms on the phenyl group of these compounds are substituted with an alkyl group, an alkoxy group, or a halogen atom; and a polypropylene glycol alkyl aryl ether for which the ethyleneoxy group in the polyalkylene glycol alkyl aryl ether is substituted with a propyleneoxy group, and a polybutylene glycol alkyl aryl ether for which the ethyleneoxy group in the polyalkylene glycol alkyl aryl ether is substituted with a butyleneoxy group.

Examples of the polyalkylene glycol diaryl ethers include diethylene glycol diphenyl ether, triethylene glycol diphenyl ether, tetraethylene glycol diphenyl ether, or a polyethylene glycol diaryl ether for which some of the hydrogen atoms on the phenyl group of these compounds are substituted with an alkyl group, an alkoxy group, or a halogen atom; and a polypropylene glycol diaryl ether for which the ethyleneoxy group in the polyalkylene glycol diaryl ether is substituted with a propyleneoxy group, and a polybutylene glycol diaryl ether for which the ethyleneoxy group in the polyalkylene glycol diaryl ether is substituted with a butyleneoxy group.

Among these, from perspective of thermal degradation being less likely to occur, a polyalkylene glycol dialkyl ether is preferable, and tetraethylene glycol dibutyl ether, triethylene glycol butyloctyl ether, diethylene glycol dibutyl ether, and diethylene glycol butyl-2-chlorophenyl ether are more preferable, and from the perspective of the glycolide recovery ratio, tetraethylene glycol dibutyl ether and triethylene glycol butyloctyl ether are even more preferable.

The addition amount of the organic solvent is, for example, preferably from 30 to 5000 parts by mass, more preferably from 50 to 2000 parts by mass, and even more preferably from 100 to 1000 parts by mass, per 100 parts by mass of the glycolic acid oligomer.

Furthermore, a solubilizing agent may be further added as necessary to increase the solubility of the glycolic acid oligomer in the organic solvent.

### Solubilizing Agent

The solubilizing agent is preferably a non-basic organic compound having a boiling point of 180°C or higher, such as a monohydric alcohol, a polyhydric alcohol, a phenol, a monovalent aliphatic carboxylic acid, a polyvalent aliphatic carboxylic acid, an aliphatic amide, an aliphatic imide, or a sulfonic acid. Among these, from the perspective of being able to easily obtain an effect of a solubilizing agent, a monohydric alcohol and a polyhydric alcohol are preferable.

The boiling point of the monohydric or polyhydric alcohol is preferably 200°C or higher, more preferably 230°C or higher, and particularly preferably 250°C or higher.

Such monohydric alcohols are preferably polyalkylene glycol monoethers represented by Formula (2) below.
[Chemical Formula 2]

HO-(R¹-O)q-X¹ (2)

**In** Formula (2), R¹ denotes a methylene group or a linear or branched alkylene group having from 2 to 8 carbons. X¹ denotes a hydrocarbon group. The hydrocarbon group is preferably an alkyl group. q is an integer of 1 or greater, and when q is 2 or greater, the plurality of R¹ moieties may be mutually the same or different.

Examples of polyalkylene glycol monoethers include polyethylene glycol monoethers such as polyethylene glycol monomethyl ether, polyethylene glycol monoethyl ether, polyethylene glycol monopropyl ether, polyethylene glycol monobutyl ether, polyethylene glycol monohexyl ether, polyethylene glycol monooctyl ether, polyethylene glycol monodecyl ether, and polyethylene glycol monolauryl ether; a polypropylene glycol monoether for which an ethyleneoxy group in the polyethylene glycol monoether is substituted with a propyleneoxy group, and a polybutylene glycol monoether for which an ethyleneoxy group in the polyethylene glycol monoether is substituted with a butyleneoxy group. Among these, a polyalkylene glycol monoether having from 1 to 18 and preferably from 6 to 18 carbons in the alkyl group included in the ether group is preferable, and a polyethylene glycol monoalkyl ether such as triethylene glycol monooctyl ether is more preferable.

Since the polyalkylene glycol monoether can increase the solubility of the glycolic acid oligomer, the use of a polyalkylene glycol monoether as a solubilizing agent facilitates a more rapid advancement of the depolymerization reaction of the glycolic acid oligomer.

Polyalkylene glycols represented by Formula (3) below are preferable as the polyhydric alcohols.
[Chemical Formula 3]

HO-(R²-O)r -H (3)

In Formula (3), R² denotes a methylene group or a linear or branched alkylene group having from 2 to 8 carbons. r is an integer of 1 or greater, and when r is 2 or greater, the plurality of R² moieties may be mutually the same or different.

Examples of polyalkylene glycols include polyethylene glycol, polypropylene glycol, and polybutylene glycol.

The addition amount of the solubilizing agent is preferably from 0.1 to 500 parts by mass, and more preferably from 1 to 300 parts by mass, per 100 parts by mass of the glycolic acid oligomer. When the addition amount of the solubilizing agent is a certain amount or greater, the solubility of the glycolic acid oligomer in the organic solvent can be sufficiently enhanced, and when the addition amount is a certain amount or less, the cost required to recover the solubilizing agent can be reduced.

Next, while the obtained solution is heated under normal pressure or under reduced pressure, the glycolic acid oligomer is depolymerized.

The heating temperature during the depolymerization reaction (depolymerization temperature) may be equal to or greater than the temperature at which depolymerization of the glycolic acid oligomer occurs, and while the heating temperature depends on the degree of depressurization, the type of high boiling point organic solvent, and the like, the heating temperature is generally at least 200°C, preferably from 200°C to 350°C, more preferably from 210°C to 310°C, even more preferably from 220°C to 300°C, and yet even more preferably from 230°C to 290°C.

Heating during the depolymerization reaction is preferably performed under normal pressure or under reduced pressure, and is preferably performed under a reduced pressure from 0.1 kPa to 90 kPa. This is because the depolymerization reaction temperature decreases as the pressure is reduced, and therefore a lower pressure facilitates a reduction in the heating temperature, and the recovery ratio of the solvent is increased. The degree of depressurization is preferably from 1 kPa to 60 kPa, more preferably from 1.5 kPa to 40 kPa, and even more preferably from 2 kPa to 30 kPa.

Next, the produced glycolide is distilled out of the depolymerization reaction system along with the organic solvent. By distilling out the produced glycolide along with the organic solvent, adherence and accumulation of the glycolide on wall surfaces of the reaction vessel and lines can be prevented.

Glycolide is then recovered from the obtained distillate. Specifically, the distillate is cooled and phase separated, and glycolide is precipitated. The precipitated glycolide is separated and recovered from the mother liquor by a method such as filtration, centrifugal sedimentation, or decantation.

The mother liquor from which the glycolide has been separated may be recycled and used as is without purification, or may be recycled and used after being treated with activated carbon and filtered and purified, or after being purified through distillation once again.

When the glycolide is distilled out together with the organic solvent, the volume of the depolymerization reaction system decreases. In contrast, the depolymerization reaction can be performed continuously or repeatedly for a long period of time by adding, to the depolymerization reaction system, a glycolic acid oligomer and an organic solvent in an amount equivalent to the amount that was distilled away.

As described above, in the present invention, metal titanium is added to the aqueous glycolic acid solution to carry out a dehydrating polycondensation reaction and a depolymerization reaction. As a result, the production rate of glycolide can be dramatically increased.

### 2. Glycolide

The glycolide (also referred to as crude glycolide) obtained by the production method of an embodiment of the present invention is preferably high in purity. Specifically, the purity of the glycolide is preferably not less than 99.0%, more preferably not less than 99.3%, and even more preferably not less than 99.5%. The purity of glycolide can be measured by gas chromatography (GC) using 4-chlorobenzophenone as the internal standard.

Thus, according to the glycolide production method of an embodiment of the present invention, high purity glycolide can be obtained at a high production rate.

### Examples

The present invention will be described in further detail below with reference to examples. The scope of the present invention is not to be construed as being limited by these examples and is defined by the current set of claims. Examples which do not fall under the scope of the claims are provided as reference.

### 1. First Embodiment (according to the present invention)

### Example 1

A separable flask having a volume of 1 L was charged with 1.3 kg of an aqueous solution of 70 mass% glycolic acid (available from The Chemours Company), and 19.5 mg of titanium powder (29 ppm with respect to the glycolic acid, average particle size of 24 µm) was added thereto (step 1 described above). Note that the average particle size of the titanium powder was measured from a volume average arithmetic mean using a particle size distribution measurement device.

Next, the mixture was heated under stirring at normal pressure to increase the temperature from room temperature to 215°C, and a polycondensation reaction was carried out while distilling away water that was produced. Subsequently, the pressure inside the flask was gradually reduced from normal pressure to 3 kPa, after which the contents in the flask were heated at 215°C for 3 hours, low boiling point substances such as unreacted raw materials were distilled away, and a glycolic acid oligomer (weight average molecular weight Mw of from 22000 to 24000, melting point of from 210 to 220°C) was obtained (step 2 described above).

Next, 126 g of the obtained glycolic acid oligomer, 130 g of tetraethylene glycol dibutyl ether (high boiling point organic solvent), and 100 g of triethylene glycol monooctyl ether (solubilizing agent) were added to a container having a volume of 0.5 L, and then heated to 235°C, and the reaction system was formed into a homogeneous solution. While this reaction system was heated at a temperature of 235°C under stirring at a speed of 170 rpm, a depolymerization reaction was carried out for 12 hours under a reduced pressure of 3 kPa (step 3 described above). During the reaction, every one hour, tetraethylene glycol dibutyl ether and crude glycolide were co-distilled, the crude glycolide was separated and recovered from the co-distillate, and the mass was measured.

Along with the recovery of crude glycolide every one hour, glycolic acid oligomer in an amount equivalent to the mass (one-fold amount) of the recovered crude glycolide was newly fed into the reaction system. The amount of crude glycolide recovered per hour was arithmetically averaged to obtain the production rate (g/h) of the crude glycolide.

### Example 2

The crude glycolide production rate was determined in the same manner as in Example 1 with the exception that the addition amount of titanium powder was changed to 325 mg (357 ppm relative to glycolic acid).

### Comparative Example 1

The crude glycolide production rate was determined in the same manner as in Example 1 with the exception that titanium powder was not added.

The evaluation results for each of Examples 1 and 2 and Comparative Example 1 are shown in Table 1.

**Table 1**

| | Added material | Addition Amount of Titanium with respect to Glycolic Acid (ppm) | Crude Glycolide Production Rate (g/h) |
|---|---|---|---|
| Example 1 | Titanium powder | 21 | 24.5 |
| Example 2 | Titanium powder | 357 | 23.6 |
| Comparative Example 1 | - | - | 12.9 |

As shown in Table 1, in Examples 1 and 2 in which titanium powder was added, the production rate of crude glycolide was higher than that of Comparative Example 1 in which titanium powder was not added.

### 2. Second Embodiment (not according to the present invention)

### (1) Preparation of Glycolic Acid Oligomers

### Synthesis Example 1

A reaction vessel made of titanium and having a volume of 18 m³ was charged with 18000 kg (40000 lbs) of an aqueous solution of 70% glycolic acid (available from The Chemours Company). The solution was then heated from room temperature to 115°C under stirring at normal pressure, and then maintained at a temperature of from 50°C to 115°C for 7 days. Note that the boiling point of an aqueous glycolic acid solution typically increases as the concentration of glycolic acid increases. Specifically, since the boiling point of the aqueous solution of 70% glycolic acid is 115°C, the boiling point of the aqueous glycolic acid solution after reaching 115°C becomes higher than 115°C (in association with the increase in concentration). Thus, maintaining a temperature of from 50°C to 115°C after reaching 115°C means maintaining a temperature that is always lower than the boiling point (at the concentration at that time). Next, the solution was further heated to 125°C, and heating and stirring were performed for three days while water was distilled away. The amount of titanium dissolved in the solution at this time was 356 ppm relative to the total mass of the glycolic acid.

Next, the solution was further heated to 215°C, and a dehydrating polycondensation reaction was carried out while distilling away the water that was produced. Subsequently, the pressure inside the reaction vessel was gradually reduced from normal pressure to 3 kPa, after which the contents in the reaction vessel were heated at 215°C for 3 hours, low boiling point substances such as unreacted raw materials were distilled away, and a glycolic acid oligomer was obtained.

### Synthesis Example 2

A separable flask having a volume of 1 L was charged with 1.3 kg of an aqueous solution of 70% glycolic acid (available from The Chemours Company). Next, the solution was heated from room temperature to 215°C under stirring at normal pressure, and a polycondensation reaction was carried out while distilling away the water that was produced. In this heating process, after a temperature of 115°C (the boiling point of the aqueous solution of 70% glycolic acid) was reached, the temperature of the aqueous glycolic acid solution was always the same as the boiling point (at the concentration at that time). The stirring speed was set to 170 rpm.

Subsequently, the pressure inside the reaction vessel was gradually reduced from normal pressure to 3 kPa, after which the contents in the reaction vessel were heated at 215°C for 3 hours to distill away the low boiling substances such as unreacted raw materials, and a glycolic acid oligomer was obtained.

### Synthesis Example 3

A reaction vessel made of titanium and having a volume of 18 m³ was charged with 18000 kg (40000 lbs) of an aqueous solution of 70% glycolic acid (available from The Chemours Company). Next, the mixture was heated for approximately 24 hours under stirring at normal pressure to increase the temperature from room temperature to 215°C, and a polycondensation reaction was carried out while distilling away the water that was produced. In this heating process, after a temperature of 115°C (the boiling point of the aqueous solution of 70% glycolic acid) was reached, the temperature of the aqueous glycolic acid solution was always the same as the boiling point (at the concentration at that time). The amount of titanium dissolved in the solution at this time was 4.7 ppm relative to the total mass of glycolic acid.

Subsequently, the pressure inside the reaction vessel was gradually reduced from normal pressure to 3 kPa, after which the contents in the reaction vessel were heated at 215°C for 3 hours to distill away the low boiling substances such as unreacted raw materials, and a glycolic acid oligomer was obtained.

The preparation conditions for Synthesis Examples 1 to 3 are summarized in Table 2.

**[Table 2]**

| | Reaction Vessel Material | Temperature Profile (Normal Pressure Process) |
|---|---|---|
| Synthesis Example 1 | Titanium | Normal temperature → 50 to 115°C (7 days) → 125°C (3 days) → 215°C |
| Synthesis Example 2 | Glass | Normal temperature → 215°C |
| Synthesis Example 3 | Titanium | Normal temperature → 215°C |

### (2) Glycolide Preparation

### Example 3 (not according to the present invention)

A flask having a volume of 0.5 L was charged with 120 g of the glycolic acid oligomer obtained in Synthesis Example 1, 130 g of tetraethylene glycol dibutyl ether, and 100 g of octyltriethylene glycol, after which the contents were heated to 235°C, and the reaction system was formed into a homogeneous solution.

Next, the pressure of the reaction system was reduced to 3 kPa, and a depolymerization reaction was performed for 10 hours while heating and stirring at a temperature of 235°C. During the reaction, every one hour, tetraethylene glycol dibutyl ether and crude glycolide were co-distilled, the crude glycolide was separated and recovered from the co-distillate, and the mass was measured. Along with the recovery of crude glycolide every one hour, glycolic acid oligomer of the same mass as the recovered crude glycolide was newly fed into the reaction system. The amount of crude glycolide recovered per hour was arithmetically averaged to obtain the production rate (g/h) of the crude glycolide.

### Example 4 (not according to the present invention)

A reaction vessel made of SUS and having a volume of 116 m³ was charged with 10800 kg of the glycolic acid oligomer obtained in Synthesis Example 1, 10800 kg of tetraethylene glycol dibutyl ether, and 10800 kg of octyltriethylene glycol, after which the contents were heated to 235°C, and the reaction system was formed into a homogeneous solution.

Next, the pressure of the reaction system was reduced to 3 kPa, and a depolymerization reaction was performed for 240 hours while heating and stirring at a temperature of 235°C. During the reaction, every one hour, tetraethylene glycol dibutyl ether and crude glycolide were co-distilled, and the production amount (kg/h) of the crude glycolide was confirmed using a flow meter.

### Comparative Example 2

Glycolide was produced in the same manner as in Example 3 with the exception that the glycolic acid oligomer obtained in Synthesis Example 2 was used, and the production rate (g/h) of the crude glycolide was calculated.

### Comparative Example 3

Glycolide was produced in the same manner as in Example 4 with the exception that the glycolic acid oligomer obtained in Synthesis Example 3 was used, and the production rate (kg/h) of crude glycolide was calculated.

The evaluation results of Examples 3 and 4 and Comparative Examples 2 and 3 are shown in Table 3.

**[Table 3]**

| | Glycolic Acid Oligomer | Crude Glycolide Production Rate | Scale (Depolymerization) |
|---|---|---|---|
| Example 3 | Synthesis Example 1 | 21.3 (g/h) | Lab level |
| Example 4 | | 301 (kg/h) | Plant level |
| Comparative Example 2 | Synthesis Example 2 | 17.6 (g/h) | Lab level |
| Comparative Example 3 | Synthesis Example 3 | 246 (kg/h) | Plant level |

As indicated in Table 3, it is clear that the production rate of crude glycolide was higher in Example 3 in which the glycolic acid oligomer obtained in Synthesis Example 1 was used, than in Comparative Example 2, in which the glycolic acid oligomer obtained in Synthesis Example 2 was used. It is presumed that the reason for this is that in Synthesis Example 2, there was no elution of the active component from the reaction vessel made of glass, whereas in Synthesis Example 1, titanium ions (which are an active component) were eluted from the reaction vessel made of titanium, dispersed well in the obtained glycolic acid oligomer, and acted as a catalyst.

It is also clear that the production rate of crude glycolide was higher in Example 4, in which the glycolic acid oligomer of Synthesis Example 1 (which had undergone a step of maintaining the temperature and stirring in a reaction vessel made of titanium) was used, than in Comparative Example 3, in which the glycolic acid oligomer of Synthesis Example 3 (which did not undergo a step of maintaining the temperature and stirring for a certain period of time or longer within a reaction vessel made of titanium). It is presumed that the reason for this is that titanium ions eluted from the titanium reaction vessel were better dispersed in the glycolic acid oligomer obtained in Synthesis Example 1 than in the glycolic acid oligomer obtained in Synthesis Example 3, and the dispersed titanium ions acted as a catalyst.

The present application claims priority to JP 2018-052293 and JP 2018-052289 filed on March 20, 2018.

### Industrial Applicability

According to the present invention, a glycolide production method capable of sufficiently increasing the production rate of glycolide can be provided.

## Claims

1. A glycolide production method comprising:
adding metal titanium to an aqueous glycolic acid solution;
subjecting glycolic acid contained in the aqueous glycolic acid solution to which the metal titanium is added, to dehydrating polycondensation to obtain a glycolic acid oligomer; and
heating and depolymerizing the glycolic acid oligomer to obtain glycolide under the existence of a titanium ion generated from the metal titanium; wherein the metal titanium is a titanium powder, and an addition amount of the metal titanium is from 5 ppm to 1000 ppm relative to a total mass of the glycolic acid.

2. The glycolide production method according to claim 1, wherein an average particle size of the titanium powder is equal to or less than 100 µm.

3. The glycolide production method according to any one of claims 1 or 2, wherein a dehydrating polycondensation temperature is from 50°C to 300°C.

4. The glycolide production method according to any one of claims 1 to 3, wherein the depolymerization is performed in an organic solvent.

5. The glycolide production method according to claim 4, wherein the organic solvent comprises a polyalkylene glycol ether represented by Formula (1) below: [Chemical Formula 1]
X-O-(-R-O-)p -Y (1)
where R denotes a methylene group or a linear or branched alkylene group having from 2 to 8 carbons,
X and Y each independently denote an alkyl group or an aryl group having from 2 to 20 carbons,
p is an integer from 1 to 5, and
when p is 2 or greater, a plurality of R moieties may be the same or different.

## Patentansprüche

1. Glycolid-Herstellungsverfahren, umfassend:
Hinzufügen von metallischem Titan zu einer wässrigen Glykolsäurelösung;
Unterziehen der Glykolsäure, die in der wässrigen Glykolsäurelösung enthalten ist, zu der das metallische Titan hinzugefügt ist, einer dehydrierenden Polykondensation, um ein Glykolsäureoligomer zu erhalten; und
Erhitzen und Depolymerisieren des Glykolsäureoligomers, um Glykolid unter der Anwesenheit eines Titanions zu erhalten, das aus dem metallischen Titan erzeugt wird; wobei das metallische Titan ein Titanpulver ist und eine Zusatzmenge des metallischen Titans von 5 ppm bis 1000 ppm relativ zu einer Gesamtmasse der Glykolsäure beträgt.

2. Glycolid-Herstellungsverfahren nach Anspruch 1, wobei eine durchschnittliche Partikelgröße des Titanpulvers gleich oder kleiner als 100 µm ist.

3. Glycolid-Herstellungsverfahren nach einem der Ansprüche 1 oder 2, wobei eine Temperatur der dehydrierten Polykondensation von 50 °C bis 300 °C beträgt.

4. Glycolid-Herstellungsverfahren nach einem der Ansprüche 1 bis 3, wobei die Depolymerisation in einem organischen Lösungsmittel durchgeführt wird.

5. Glycolid-Herstellungsverfahren nach Anspruch 4, wobei das organische Lösungsmittel einen Polyalkylenglycolether umfasst, der durch die nachstehende Formel (1) dargestellt wird:
[Chemische Formel 1]
X-O-(-R-O-)p-Y (1)
wobei R eine Methylengruppe oder eine lineare oder verzweigte Alkylengruppe bezeichnet, die von 2 bis 8 Kohlenstoffatome aufweist,
X und Y jeweils unabhängig eine Alkylgruppe oder eine Arylgruppe bezeichnen, die von 2 bis 20 Kohlenstoffatome aufweisen,
p eine ganze Zahl von 1 bis 5 ist und
wenn p 2 oder mehr ist, eine Vielzahl von R-Teilen gleich oder unterschiedlich sein können.

## Revendications

1. Procédé de production de glycolide comprenant :
l'ajout de métal titane à une solution aqueuse d'acide glycolique ;
la soumission de l'acide glycolique contenu dans la solution aqueuse d'acide glycolique à laquelle le titane métallique est ajouté, à une polycondensation déshydratante pour obtenir un oligomère d'acide glycolique ; et
le chauffage et la dépolymérisation de l'oligomère d'acide glycolique pour obtenir le glycolide sous l'effet d'un ion titane généré à partir du titane métallique ; dans lequel le titane métallique est une poudre de titane, et la quantité de titane métallique ajoutée est comprise entre 5 ppm et 1 000 ppm par rapport à la masse totale de l'acide glycolique.

2. Procédé de production de glycolide selon la revendication 1, dans lequel une taille moyenne des particules de la poudre de titane est égale ou inférieure à 100 µm.

3. Procédé de production de glycolide selon l'une quelconque des revendications 1 ou 2, dans lequel la température de polycondensation déshydratante est comprise entre 50 °C et 300 °C.

4. Procédé de production de glycolide selon l'une quelconque des revendications 1 à 3, dans lequel la dépolymérisation est effectuée dans un solvant organique.

5. Procédé de production de glycolide selon la revendication 4, dans lequel le solvant organique comprend un éther de polyalkylène glycol représenté par la formule (1) ci-dessous :
[Formule chimique 1]
X-O-(-R-O-)p-Y (1)
où R désigne un groupe méthylène ou un groupe alkylène linéaire ou ramifié ayant de 2 à 8 carbones,
X et Y désignent chacun indépendamment un groupe alkyle ou un groupe aryle ayant de 2 à 20 carbones,
p est un nombre entier compris entre 1 et 5, et
lorsque p est égal ou supérieur à 2, plusieurs fractions R peuvent être identiques ou différentes.
